# DEMANDE DE BREVET EUROPEEN

(11) **EP 1 449 517 A1**
(43) Date de publication de la demande: **25.08.2004**
(21) Numéro de dépôt: 04300076.9
(22) Date de dépôt: 12.02.2004
(51) Int. Cl.: A61K 7/48, A61K 31/198, A61P 3/04

(54) **Utilisation de N-Lauroyl aminoacides comme actif cosmétique et pharmaceutique aminissant**

(30) Priorité: 21.02.2003 FR 0302161
(71) Demandeur: SOCIETE D'EXPLOITATION DE PRODUITS POUR LES INDUSTRIES CHIMIQUES, S.E.P.P.I.C., 75321 Paris Cédex 07 (FR)
(72) Inventeur: Garcia, Christine, 81100 Castres (FR)
(74) Mandataire: Conan, Philippe Claude

(57) **Abrégé**

Utilisation d'un composé de formule (I) : 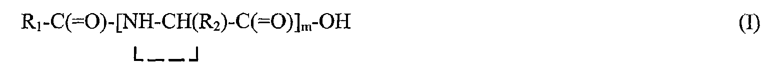 dans laquelle R₁ représente un radical hydrocarboné comportant 11 atomes de carbone, R₂ représente la chaîne caractérisante d'un acide aminé et m est compris entre 1 et 50, ou d'un mélange desdits composés de formule (I), comme actif amincissant. Procédé de traitement non thérapeutique mettant en oeuvre ledit composé et utilisation dudit composé pour préparer un médicament à activité lipolytique, destiné à induire l'amincissement du corps humain.

## Description

La présente invention a pour objet une nouvelle utilisation d'actifs cosmétiques pour amincir le corps humain.

Une partie de la graisse du corps humain est stockée sous forme de triglycérides, dans des cellules du tissu gras du derme, appelés adipocytes. L'amincissement rend compte d'une diminution de la graisse stockée dans les adipocytes. Ce processus nécessite une étape prioritaire qui a lieu à l'intérieur de ces cellules et qui consiste en l'hydrolyse des triglycérides en acides gras et glycérol. Ce phénomène s'appelle la lipolyse.

La plupart des formulations cosmétiques amincissantes commercialisées aujourd'hui, contiennent au moins un composé possédant une activité lipolytique. Le plus fréquemment utilisé est la caféine mais la théophylline est aussi connue pour posséder une telle propriété.

A l'occasion de leur recherche de nouveaux actifs à activité lipolytique qui aient une meilleure compatibilité avec la peau que ceux de l'état de la technique, les inventeurs ont mis en évidence que certains dérivés N-acylés d'acides aminés connus pour leur propriété apaisante possédaient aussi une propriété lipolytique plus efficace que celle de la caféine

C'est pourquoi, selon un premier aspect, l'invention a pour objet, l'utilisation d'un composé de formule (I) : 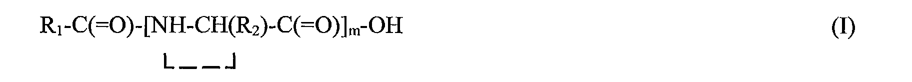 dans laquelle R₁ représente un radical hydrocarboné aliphatique, saturé ou insaturé, linéaire ou ramifié, comportant 11 atomes de carbone, R₂ représente la chaîne caractérisante d'un acide aminé et m est compris entre 1 et 50, ou d'un mélange desdits composés de formule (I), comme actif amincissant, dans une composition contenant un milieu cosmétiquement acceptable.

Le composé de formule (I) telle que définie ci-dessus, peut être sous forme d'acide libre ou sous forme partiellement ou totalement salifiée. Lorsque le composé de formule (I) est sous forme salifiée, il s'agit notamment de sels alcalins tels que les sels de sodium, de potassium ou de lithium, de sels alcalino-terreux tels que les sels de calcium, de magnésium ou de strontium ; de sel d'ammonium ou de sel d'un aminoalcool comme le sel de (2-hydroxy éthyl) ammonium. Il peut aussi s'agir de sels métalliques tels que les sels divalents de zinc ou de manganèse, les sels trivalents de fer, de lanthane, de cérium ou d'aluminium. De façon générale, le taux de salification du composé de formule (I) telle que définie ci dessus, dépendra en autre de son pK_{A} et de la concentration en sels de la composition dans laquelle il est incorporé, le composé de formule (I) sera présent.

Dans l'exposé suivant, par composé de formule (I), on entend composé de formule (I) sous forme libre ou sous forme partiellement ou totalement salifiée.

L'expression "chaîne caractérisante" utilisée pour définir le radical R₂, désigne la chaîne principale non fonctionnelle de l'acide aminé considéré. Ainsi, pour un acide aminé représenté par la formule générale (IIIa) :

H₂N-CH(R₂)-C(=O)-OH (IIIa),

comme pour un acide aminé cyclique représenté par la formule (IIIb) : 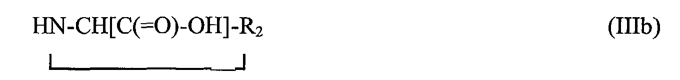 la chaîne caractérisante sera la chaîne représentée par R₂.

R₂ représente notamment la chaîne caractérisante d'un acide aminé choisi parmi la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la glutamine, la cystéine, la cystine, la méthionine, l'hydroxy proline, l'hydroxy lysine, la sarcosine ou l'ornithine.

L'invention a principalement pour objet, l'utilisation d'un composé de formule (I) telle que définie précédemment, dans laquelle, dans au moins un des restes :

- HN-CH(R₂)-C(=O)- (IIIa),

ou 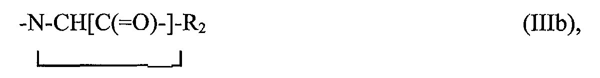 R₂ représente la chaîne caractérisante de la glycine, de l'alanine, de l'acide aspartique, de l'acide glutamique, ou de la sarcosine.

L'invention a plus particulièrement pour objet, l'utilisation d'un composé de formule (I), telle que définie précédemment dans laquelle m est un nombre décimal compris entre 1 et 10 et il est de préférence inférieur à 5.

Selon un aspect tout particulier de la présente invention, dans la formule (I) telle que définie précédemment, m est inférieur ou égal à 2 et est plus particulièrement inférieur ou égal à 1,4.

Selon un autre aspect tout particulier de la présente invention, dans la formule (I) telle que définie précédemment, m est égal à 1.

Selon une autre variante particulière de la présente invention, on utilise un seul composé de formule (I) telle que définie précédemment, dans la composition contenant le milieu cosmétiquement acceptable.

Selon une autre variante particulière de la présente invention, on utilise un mélange de composés de formule (I) telle que définie précédemment.

Les composés de formules (I) sont généralement obtenus par N-acylation de composés de formules (IIIa) ou (IIIb), telles que définies précédemment ou de leurs sels.

Lorsqu'il s'agit d'un mélange de composés de formules (I), il est par exemple obtenu par N-acylation du mélange d'acides aminés résultant de l'hydrolyse totale ou partielle de protéines de toutes origines.

Ces protéines peuvent être d'origine animale, telles que, par exemple, le collagène, l'élastine, la protéine de chair de poisson, la gélatine de poissons, la kératine ou la caséine, d'origine végétale, comme les protéines de céréales, de fleurs ou de fruits, telles que par exemple, les protéines issues du soja, du tournesol, de l'avoine, du blé, du maïs, de l'orge, de la pomme de terre, du lupin, de la féverole, de l'amande douce, du kiwi, de la mangue ou de la pomme ; il peut s'agir aussi de protéines obtenues à partir de chorelles (algues unicellulaires), d'algues roses, de levures ou de la soie.

Cette hydrolyse est réalisée par exemple, par chauffage à des températures comprises entre 60 et 130°C d'une protéine placée dans un milieu acide ou alcalin.

Cette hydrolyse peut également être réalisée par voie enzymatique avec une protéase, couplée éventuellement à une post-hydrolyse alcaline ou acide. Quand m est supérieur à 1, R₂ représente une seule même chaîne ou bien plusieurs chaînes caractérisant différents acides aminés, selon la protéine hydrolysée et le degré d'hydrolyse.

Les aminogrammes de quelques protéines d'origine végétales sont consignés dans les tableaux suivants :

**Tableau 1**

| | Origine de la protéine (proportions en acides aminés exprimées en % pondéraux) | | | |
|---|---|---|---|---|
| | Avoine | Soja | Blé | Tournesol |
| Glycine | 6,9 | 4,2 | 3,2 | 6,2 |
| Alanine | 5,9 | 4,2 | 2,6 | 4,8 |
| Serine | 5,6 | 5,1 | 1,7 | 5,1 |
| Acide aspartique | 16,2 | 11,7 | 3,4 | 10,6 |
| Acide glutamique | 28,3 | 19,1 | 37,9 | 23,6 |
| Valine | 2,9 | 5,0 | 4,2 | 4,8 |
| Thréonine | 3,1 | 3,9 | 2,7 | 4,4 |
| Arginine | 6,6 | 7,8 | 3,7 | 8,4 |
| Lysine | 3,6 | 6,2 | 1,9 | 3,2 |
| Proline | 4,7 | 5,4 | 11,7 | 3,0 |
| Leucine | 6,4 | 8,1 | 7,1 | 6,4 |
| Phénylalanine | 1,4 | 5,0 | 5,4 | 4,3 |
| Isoleucine | 2,2 | 4,8 | 3,7 | 4,1 |
| Histidine | 1,7 | 2,6 | 2,4 | 2,0 |
| Tyrosine | 1,5 | 3,5 | 3,1 | 2,7 |
| Méthionine | 1,2 | 1,2 | 1,6 | 1,8 |
| Cystéine / Cystine | 1,9 | 1,5 | 1,9 | 1,9 |
| Tryptophane | - | 1,0 | 1,0 | 1,3 |

**Tableau 2**

| | Origine de la protéine (proportions en acides aminés exprimées en % pondéraux) | | | |
|---|---|---|---|---|
| | Lupin | Pomme de terre | Féverole | Maïs |
| Glycine | 0,9 | 4,8 | 4,0 | 2,4 |
| Alanine | 2,4 | 5,0 | 4,0 | 7,95 |
| Serine | 6,1 | 5,8 | 4,9 | 5,1 |
| Acide aspartique | 15,8 | 12,5 | 10,5 | 10,6 |
| Acide glutamique | 8,0 | 11,5 | 16,8 | 23,6 |
| Valine | 7,9 | 7,1 | 4,5 | 4,8 |
| Thréonine | 8,1 | 6,1 | 3,6 | 4,4 |
| Arginine | 16,1 | 5,0 | 9,21 | 8,4 |
| Lysine | 7,1 | 7,8 | 6,5 | 6,2 |
| Proline | - | 5,1 | 4,4 | 3,0 |
| Leucine | 7,45 | 10,4 | 7,4 | 8,1 |
| Phénylalanine | 8,6 | 6,4 | 4,4 | 4,3 |
| Isoleucine | 8,7 | 6,1 | 3,9 | 4,1 |
| Histidine | - | 2,2 | 2,6 | 2,0 |
| Tyrosine | - | 5,7 | 3,6 | 2,7 |
| Méthionine | 0,6 | 2,4 | 0,8 | 1,8 |
| Cystéine / Cystine | - | 1,6 | 1,7 | 1,9 |
| Tryptophane | 1,2 | 1,4 | 1,2 | 1,3 |
| Ornithine | 0,4 | - | - | - |

La réaction d'acylation est connue de l'homme du métier. Elle est décrite par exemple dans la demande internationale publiée sous le numéro WO 98/09611. Elle est mise en oeuvre indifféremment sur un acide aminé ou sur un mélange d'acides aminés. L'agent d'acylation consiste généralement en un dérivé activé d'un acide carboxylique de formule R₁-C(=O)-OH, dans laquelle R₁ est tel que défini précédemment, tel qu'un anhydride symétrique de cet acide, l'ester méthylique de cet acide, ou un halogénure d'acide comme le chlorure d'acide ou le bromure d'acide. Il peut aussi consister en un mélange de dérivés activés d'acides carboxyliques issus d'huiles ou graisses naturelles d'origine animales ou végétales telles que les huiles de coprah, de palmiste, de palme, de soja, de colza, de maïs, le suif de boeuf, l'huile spermaceti ou l'huile de hareng. Dans, le cadre de la présente invention on utilise de préférences les mélanges d'acides gras issus de l'huile de coprah, de l'huile de palmiste ou de l'huile de spermaceti qui contiennent une fraction majoritaire en acide dodécanoïque :

| | huile de coprah (% en poids) | huile de palmiste (% en poids) | huile de spermaceti (% en poids) |
|---|---|---|---|
| acide octanoïque ou caprylique (C₈H₁₆O₂) | 6% à 9% | 3 à 10% | - |
| acide décanoïque ou caprique (C₁₀H₂₀O₂) | 6% à 10% | 3% à 14% | 1% à 3% |
| acide dodécanoïque ou laurique (C₁₂H₂₄O₂) | 44 % à 51 % | 37% à 52% | 14 % à 38 % |
| acide tétradécanoïque ou myristique (C₁₄H₂₈O₂) | 13% à 18% | 7% à 17% | 12% à 14% |
| acide hexadécanoïque ou palmitique (C₁₆H₃₂O₂) | 8% à 10% | 2% à 9% | 8% à 10% |
| acide octadécanoïque ou stéarique (C₁₈H₃₆O₂) | 1% à 3% | 1% à 3% | 1% à 3% |
| acide octadécénoïque ou oléique (C₁₈H₃₄O₂) | 5,5 % à 7,5 % | 11 % à 23 % | 15 % à 18 % |
| acide eicosénoïque ou gadolique (C₂₀H₃₈O₂) | - | - | 5 % à 8 % |
| acide octadécadiénoïque ou linoléique (C₁₈H₃₂O₂) | < 2,5 % | 1% à 3% | - |
| autres acides | < 0,4 % | < 0, 6 % | 26 % à 34 % |

Selon un aspect particulier de la présente invention, celle-ci a pour objet, l'utilisation de N-cocoyl aminoacides comme actif amincissant, dans une composition contenant un milieu cosmétiquement acceptable.

Par N-cocoyl aminoacides, on désigne un mélange de composés de formule (I) obtenu par acylation d'un acide aminé ou d'un mélange d'acides aminé avec les dérivés activés d'acides gras issus de l'huile de coprah.

L'invention a aussi pour objet, un procédé de traitement non thérapeutique du corps humain destiné à l'amincir, caractérisé en ce que l'on y applique une composition contenant un milieu cosmétiquement acceptable et une quantité efficace d'au moins un composé de formule (I) telle que définie précédemment.

L'invention a aussi pour objet l'utilisation d'au moins un composé de formule (I), telle que définie précédemment, pour préparer un médicament à activité lipolytique, destiné à induire l'amincissement du corps humain.

Dans les compositions définies ci-dessus, le composé de formule (I), est généralement mis en oeuvre en une quantité comprise entre 0,01 % et 10 % de leur poids, plus particulièrement entre 0,1 % à 5 % de leurs poids, et tout particulièrement entre 1 % et 5 % de leurs poids.

Comme le montrent les exemples suivants, les composés mis en oeuvre dans les traitements cosmétiques ou thérapeutiques définis précédemment, se caractérisent de façon inattendue, par une activité lipolytique supérieure aux compositions de l'état de la technique. Ils sont donc de façon générale, appropriés aux traitements amincissants du corps humain.

Les compositions mises en oeuvre dans lesdits traitements, se présentent généralement sous forme de solutions aqueuses ou hydroalcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale, ou sous forme de poudre. Elles peuvent aussi être dispersées ou imprégnées sur du textile ou sur des matériaux non tissés qu'il s'agisse de lingettes, de serviettes en papier ou de vêtements.

Les compositions mises en oeuvre dans lesdits traitements, sont administrées au sujet sous les formes classiques utilisées en cosmétique et en pharmacie ; il s'agit plus particulièrement des administrations topique, orale ou parentérale.

De façon générale, les composés de formule (I), sont associés à de nombreux types d'adjuvants ou de principes actifs utilisés dans les formulations cosmétiques, qu'il s'agisse, de corps gras, de solvants organiques, d'épaississants, de gélifiants, d'adoucissants, d'antioxydants, d'opacifiants, de stabilisants, de moussants, de parfums, d'émulsionnants, ioniques ou non, de charges, de séquestrants, de chélateurs, de conservateurs, de filtres chimiques ou de filtres minéraux, d'huiles essentielles, de matières colorantes, de pigments, d'actifs hydrophiles ou lipophiles, d'humectants, par exemple la glycérine, de conservateurs, de colorants, de parfums, d'actifs cosmétiques, de filtres solaires minéraux ou organiques, de charges minérales comme les oxydes de fer, oxydes de titane et le talc, de charges synthétiques comme les nylons et les poly(méthacrylate de méthyle) réticulés ou non, d'élastomères silicone, de séricites ou d'extraits de plantes ou encore de vésicules lipidiques ou tout autre ingrédient habituellement utilisé en cosmétique.

Comme exemples d'huiles que l'on peut associer au composé de formule (I), on peut citer, les huiles minérales telles que l'huile de paraffine, l'huile de vaseline, les isoparaffines ou les huiles blanches minérales, les huiles d'origine animale, telles que le squalène ou le squalane, les huiles végétales, telles que l'huile d'amande douce, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiées par des amines, les silicones modifiées par des acides gras, les silicones modifiées par des alcools, les silicones modifiées par des alcools et des acides gras, des silicones modifiées par des groupements polyéther, des silicones époxy modifiées, des silicones modifiées par des groupements fluorés, des silicones cycliques et des silicones modifiées par des groupements alkyles.

Comme autre matière grasse que l'on peut associer à cet actif, on peut citer les alcools gras ou les acides gras.

Parmi les polymères épaississants et/ou émulsionnant utilisés dans la présente invention, il y a par exemple, les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique, de chlorure de triméthylaminoéthylacrylate, les hydrocolloïdes d'origine végétale ou biosynthétique, par exemple la gomme de xanthane, la gomme de karaya, les carraghénates, les alginates ; les silicates ; la cellulose et ses dérivés ; l'amidon et ses dérivés hydrophiles ; les polyuréthanes. Parmi les polymères de type polyélectrolytes, pouvant être mis en jeu dans la production d'une phase aqueuse gélifiée apte à être utilisée dans la préparation d'émulsions E/H contenant les composés de formule (I) objets de la présente invention, il y a par exemple, les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL^{TM} EG, SEPIGEL^{TM} 305, SIMULGEL^{TM} NS, SIMULGEL^{TM} 800 et SIMULGEL^{TM} A par la demanderesse.

Parmi les cires utilisables dans la présente invention, on peut citer par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

Parmi les émulsionnants utilisables dans la présente invention, on peut citer par exemple les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435 et 2 804 432.

Comme exemples de principe actif que l'on peut associer au composé de formule (I), on peut citer les composés ayant une action éclaircissante ou dépigmentante tels que par exemple l'arbutine, l'acide kojique, l'hydroquinone, l'acide ellagique, la vitamine C, le magnésium ascorbyl phosphate les extraits de polyphénols, les extraits de raisin, les extraits de pin, les extraits de vin, les extraits d'olives, les extraits de mare, les protéines N-acylées, les peptides N-acylés, les acides aminés N-acylés, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol), l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, la vitamine E et ses dérivés, les minéraux, les enzymes, les co-enzymes, comme, le Coenzyme Q10, les hormones ou "hormone like", les extraits de soja par exemple, la Raffermine^{TM}, les extraits de blé par exemple la Tensine^{TM} ou la Gliadine^{TM}, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action amincissante comme la caféine ou ses dérivés, les actifs ayant une activité anti-microbienne ou une action purifiante vis à vis des peaux grasses tels que le LI-PACIDE^{TM} PVB, les actifs ayant une propriété énergisante ou stimulante comme le SEPI-TONIC^{TM} M3 ou le Physiogényl^{TM} le panthénol et ses dérivés comme le SEPICAP^{TM} MP, les actifs anti-age comme le SEPILIFT^{TM} DPHP, le LIPACIDE^{TM} PVB, le SEPIVINOL^{TM}, le SEPIVITAL^{TM}, les actifs hydratants comme le SEPICALM^{TM} S, le SEPICALM^{TM} VG et le LIPACIDE^{TM} DPHP, les actifs anti-âge " anti-photo vieillissement ", les actifs protecteurs de l'intégrité de la jonction dermo-épidermique, les actifs augmentant la synthèse des composants de la matrice extracellulaire, les actifs ayant une activité amincissante, raffermissante ou drainante comme la caféine, la théophylline, l'AMPc, le thé vert, la sauge, le ginko biloba, le lierre, le marron d'inde, le bambou, le ruscus, le petit houx, la centella asiatica, la bruyère, l'ulmaine, le fucus, le romarin, la saule , des actifs créant une sensation de « chauffe » sur la peau comme les activateurs de la microcirculation cutanée (exemple des nicotinates) ou des produits créant une sensation de « fraîcheur » sur la peau (exemple du menthol et des dérivés).

Comme filtre solaire que l'on peut incorporer dans la composition selon l'invention, on peut citer tous ceux figurant dans la directive cosmétique 76/768/CEE modifiée annexe VII.

L'étude expérimentale suivante illustre l'invention sans toutefois la limiter.

### I) Evaluation in vitro de l'activité lipolytique des composés de formule (I)

### A - Dosages des acides gras libres

### (1) - But et principe de la méthode

L'expérience a pour objet de mettre en évidence, dans un modèle in vitro d'adipocytes humains isolés, l'activité lipolytique des composés mis en oeuvre. L'hydrolyse des triglycérides en acides gras non estérifiés et en glycérol est appelée lipolyse. Les triglycérides sont stockés dans les adipocytes et constituent la réserve graisseuse. Pour que cette réserve soit diminuée, ce qui est la finalité recherchée lorsque l'on utilise des produits amincissants, les triglycérides doivent être hydrolysés sous forme d'acides gras, qui eux peuvent être éliminés de la cellule. L'hydrolyse des triglycérides met en jeu une lipase hormonodépendante qui doit être phosphorylée pour être active. L'étape de phosphorylation met en jeu une kinase et l'AMPc. Une augmentation du contenu en AMPc des adipocytes est nécessaire pour favoriser l'activité de la lipase et donc la lipolyse. La méthode décrite consiste en une incubation des produits en présence d'adipocytes humains en suspension, suivie d'une mesure du taux intracellulaire en AMPc.

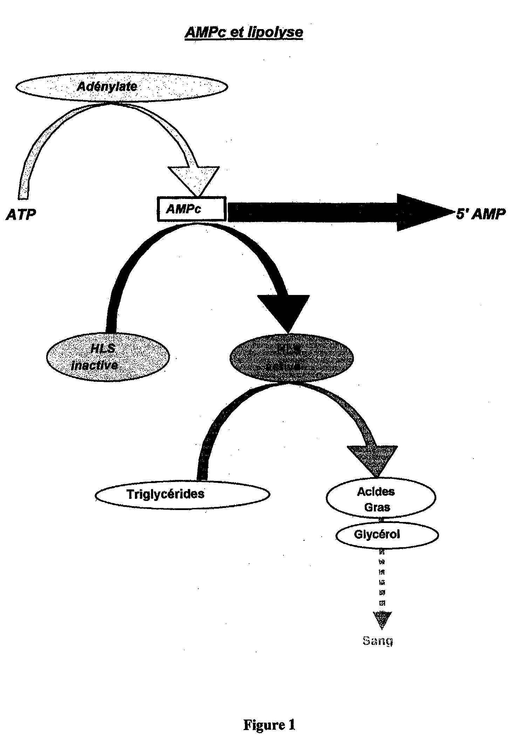

La figure 1 illustre ce mécanisme d'action : La lipase hormone-dépendante, impliquée dans l'hydrolyse des triglycérides intra-adipocytaires, doit être phosphorylée pour être active. Cette phosphorylation met en jeu l'AMPc. Il est donc important, pour stimuler la lipolyse, d'augmenter le contenu intracellulaire en AMPc soit en augmentant la production d'AMPc par stimulation de l'adénylate cyclase, soit en diminuant la dégradation d'AMPc par inhibition de la phosphodiestérase (en particulier la PDE 3 au niveau des adipocytes).

### (2) - Protocole expérimental

### (i) Modèle cellulaire :

Le test est réalisé à partir d'adipocytes humains isolés et préparés en suspension cellulaire. Les adipocytes sont isolés à partir du tissu adipeux abdominal sous-cutané récupéré lors d'opérations de chirurgies esthétiques (plasties abdominales) réalisées sur des femmes. Les cellules sont isolées à partir du tissu frais. Le tissu adipeux est isolé et dissocié par action d'une collagènase (SIGMA^{TM}, 1 mg/ml, 30 minutes à 37°C, agitation douce). La collagènase digère le tissu conjonctif présent dans le tissu adipeux. Après digestion, les cellules sont filtrées et lavées dans un milieu de culture approprié contenant le milieu MEM sans rouge de phénol, sans glutamine (SIGMA) + 2,2 mg/ml de bicarbonate de sodium (GIBCO) + 50 UI de pénicilline (BIOWHITTAKER^{TM}) + 50 µg/ml de streptomycine (BIOWHITTAKER^{TM}) + 1% (v/v) de L-glutamine (BIOWHITTAKER^{TM}) + 0,5% d'albumine sérique délipidée (SIGMA^{TM}). La suspension d'adipocytes est utilisée immédiatement après sa préparation.

### (ii) Incubation des produits avec les adipocytes

Les produits à tester sont dilués dans le milieu de culture des adipocytes. Ils sont incubés avec les cellules en suspension pendant deux heures à 37°C (250 µl de produit + 250 µl de suspension d'adipocytes).

### (3) - Evaluation des résultats

### (i) - Concentration en acides gras libres

A l'issue de l'incubation, la lyse cellulaire est contrôlée à vue, par la présence d'une couche lipidique à la surface de la suspension cellulaire. Les milieux sous-nageants sont prélevés. Les acides gras libres sont dosés par spectrophotométrie à l'aide d'un kit commercial, (kit NEFA^{TM} C, WAKO), par référence à une gamme étalon d'acides gras. L'activité lipolytique des produits est évaluée par rapport à un groupe témoin incubé en présence des adipocytes et en absence de produit. La réactivité des adipocytes est systématiquement contrôlée pour la mesure de l'activité lipolytique des produits de référence, la caféine (1,3,7-triméthyl xanthine) et la théophylline (1,3-diméthyl 2,6-dihydroxy purine). On réalise cinq dosages pour chacun des produits testés sont les suivants :
Une Composition A comprenant :

| | |
|---|---|
| Acide lauroyl glutamique | 27 % en poids |
| Acide lauroyl aspartique | 30 % en poids |
| Acide lauroyl glycine | 6 % en poids |
| Acide lauroyl alanine | 8 % en poids |
| Acide laurique | 15 % en poids |
| eau | 10 % en poids |
| Chlorure de sodium | 1 % en poids |
| propylèneglycol | 3 % en poids |

**Une Composition A'** comprenant entre 96 % et 97 % en poids de la composition A telle que définie ci-dessus et de 3 % à 4 % en poids d'aspartate de magnésium et de potassium ;
**Une composition B** comprenant environ de 30 % à 40 % en poids de matière active de N-cocoyl aminoacides ;
**Une composition C** qui est un mélange comprenant environ 75 % en poids de composition B) et environ 25 % en poids de PECOSIL^{TM} SPP 50 (100 % de potassium diméthicone copolyol panthényl phosphate) ; et
le N laurvl sarcosinate.

Les résultats des essais, exprimés par la moyenne arithmétique des cinq dosages réalisés pour chacun des produits, sont consignés dans le tableau suivant :

| | Concentration d'incubation (% poids ES) | Concentration en acides gras libres (µM) | activité lipolytique (par rapport au témoin = 100) |
|---|---|---|---|
| Témoin | - | 11,54±2,71 | 100 |
| Caféine | 0,0019 | 15,10 ±4,11 | 131 |
| Théophylline | 0,0019 | 16,53 ±4,23 | 143 |
| Composition A | 0,0001 | 21,19 ±0,85 | 184 |
| Composition A | 0,0010 | 32,67 ± 3,14 | 283 |
| Composition A' | 0,0001 | 26,37 ±1,89 | 229 |
| Composition A' | 0,0010 | 32,25 ±0,39 | 279 |
| Composition B | 0,0001 | 24,64 ± 0,21 | 213 |
| Composition B | 0,0010 | 29,46 ± 0,56 | 241 |
| Composition C | 0,0001 | 23,66 ± 1,33 | 205 |
| Composition C | 0,0010 | 23,60 ± 0,59 | 206 |
| N-lauroyl sarcosine | 0,0001 | 26,32 ± 0,68 | 228 |
| N-lauroyl sarcosine | 0,0010 | 28,82 ± 6,11 | 250 |
| ES : extrait sec | | | |

Ces résultats font apparaître que, tandis que les composés amincissants de l'état de la technique (la caféine et la théophylline) agissent sur la lipolyse avec un facteur multiplicateur de 1,3 à 1,4 par rapport au témoin, ceux selon l'invention c'est à dire comprenant au moins un composé de formule (I) telle que définie précédemment, le font avec un facteur de 1,8 à 2,8 à des concentrations plus faibles.

Ce dosage est répété en effectuant de nouveaux essais à des concentrations différentes. Les résultats sont consignés dans le tableau suivant :

| | Concentration d'incubation(% poids ES) | dosage des acides gras non estérifiés dans le sous-nageant (% par rapport au témoin) |
|---|---|---|
| Témoin | - | 100 |
| Caféine | 0,0005 | 200 |
| Caféine | 0.0025 | 240 |
| Composition A | 0,0005 | 155 |
| Composition A | 0,0025 | 190 |
| | | |

### B - Dosage de l'AMPc intracellulaire

Les adipocytes en suspension sont mis à sec par aspiration du milieu sous-nageants. Le contenu intracellulaire en AMPc est dosé par une technique EIA à l'aide d'un kit (AMERSHAM^{TM}, RPN225). Le dosage se fait contre une gamme étalon d'AMPc. L'effet des produits sur le contenu en AMPc est évalué par rapport à un groupe témoin incubé en présence des adipocytes et en absence de produit. La réactivité des adipocytes est systématiquement contrôlée par la mesure de l'effet d'un produit de référence, la caféine.

Les résultats des essais, exprimés en contenu intracellulaire en AMPc par rapport au témoin, sont consignés dans le tableau suivant :

| | Concentration d'incubation (% poids ES) | Contenu intracellulaire en AMPc (% par rapport au témoin) |
|---|---|---|
| Témoin | - | 100 |
| Caféine | 0,0005 | 200 |
| Caféine | 0.0025 | 450 |
| Composition A | 0,0005 | 220 |
| Composition A | 0,0025 | 250 |

### C - Mesure de l'activité de la phosphodiestérase de type 3

### (1) - Principe de la méthode

Les phosphodiestérases (PDE), sont des enzymes qui dégradent l'AMPc en 5'AMP. La phosphodiestérase de type 3 ( PDE 3) est présente au niveau des adipocytes humains,. L'inhibition de cette enzyme permet d'augmenter le contenu intracellulaire en AMPc et donc d'activer la lipolyse. Le test est fondé sur une évaluation biochimique à partir d'une enzyme purifiée.

### (2) - Protocole expérimental - résultats

La phosphodiestérase de type 3, isolée de plaquettes humaines, est incubée en présence d'AMPc marqué au tritium, d'un excès d'AMPc non marqué et en présence du produit à tester ou sans produit (témoin). L'incubation est réalisée à 30°C pendant 30 minutes. A l'issue de l'incubation, la quantité de 5'AMP marqué au tritium produit est quantifiée à l'aide d'un compteur de radioactivité. La réactivité du système enzymatique est systématiquement contrôlée par la mesure de l'effet d'un produit de référence, le milrinone. Les résultats sont consignés dans le tableau suivant en pourcentage d'inhibition de l'activité de la phosphodiestérase 3 (PDE 3).

| | Concentration d'incubation (en % ES) | Pourcentage d'inhibition de l'activité PDE 3 : |
|---|---|---|
| Caféine | 0,001 | 20 % |
| Caféine | 0,01 | 70 % |
| Composition A | 0,001 | 0 % |
| Composition A | 0,01 | 60 % |

### D - Mesure de l'activité de la lipase

### (1) - Principe de la méthode

La lipase est l'enzyme qui dégrade les triglycérides en acides gras et glycérol selon le schéma réactionnel suivant : 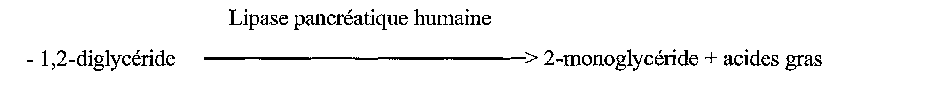 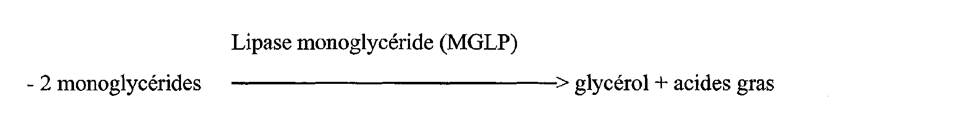 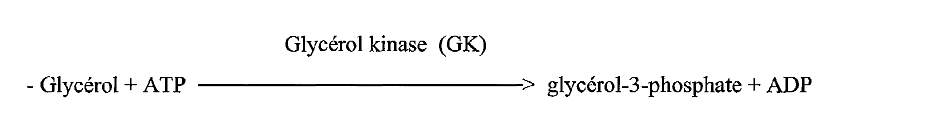 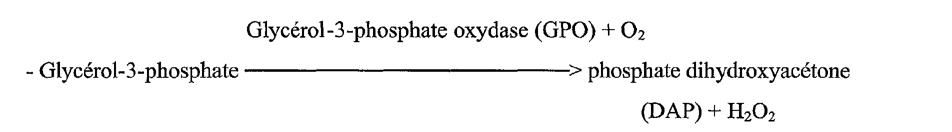 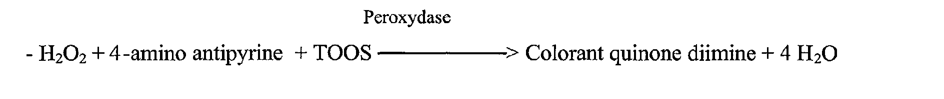 TOOS : sodium-N-éthyl N-(2-hydroxy-3-sulfopropyl)-3-toluidine

A l'intérieur des adipocytes, les acides gras libres peuvent ainsi être déstockés. L'objectif de ce test est de mettre en évidence un effet de stimulation de la lipase. Il est basé sur un test enzymatique, à partir d'une enzyme purifiée.

### (2) - Protocole expérimental - résultats :

Une lipase pancréatique humaine (SIGMA, kit LIPASE-PS^{TM}) est incubée en présence d'un di-glycéride et d'un système réactionnel pour produire une quinone diimine colorée dosable par spectrophotométrie (550 nm). La mesure de l'absorbance à 550nm est directement proportionnelle à l'activité de la lipase.

Tous les réactifs sont fournis dans le kit LIPASE-PS^{TM} (SIGMA). Le produit est incubé, avec le mélange réactionnel, pendant 8 minutes à 37°C.

Le système réactionnel mis en oeuvre est composé de :

| | |
|---|---|
| 1,2-Diglycéride | 1,1 mM |
| TOOS | 2,0 mM |
| ATP | 0,66 mM |
| MGLP (microbienne) | 860 U/l |
| GK (microbienne) | 1340 U/l |
| GPO (microbienne) | 40 000 U/l |
| Peroxydase (raifort) | 1340 U/l |
| Co-lipase (porc) | 40 000 U/l |

Les résultats sont consignés dans le tableau suivant, en pourcentage d'activation de la lipase par rapport au témoin.

| | Concentration d'incubation (en % ES) | Pourcentage d'activation de la lipase |
|---|---|---|
| Caféine | 0,01 | 0 % |
| Caféine | 0,1 | 10 % |
| Composition A | 0,01 | 25 % |
| Composition A | 0,1 | 140 % |

### E - Mesure de l'activité de la lipoprotéine lipase

### (1) - Principe de la méthode

La lipoprotéine lipase (LPL) est une enzyme rencontrée dans le compartiment extracellulaire des adipocytes. Elle assure l'hydrolyse des triglycérides contenus dans les chylomicrons en acides gras et glycérol. Ces derniers peuvent ainsi pénétrer dans les adipocytes pour y être stockés. L'objectif de ce test est de mettre en évidence un effet d'inhibition de la lipoprotéine lipase. Il est basé sur un test enzymatique, à partir d'une enzyme purifiée.

### (2) - Protocole expérimental ; résultats

Une lipoprotéine lipase (SIGMA, pseudomonase species) est incubée en présence d'un substrat, le p-nitrophényl butyrate (PNPB), qui absorbe la lumière à 400 nm après hydrolyse.

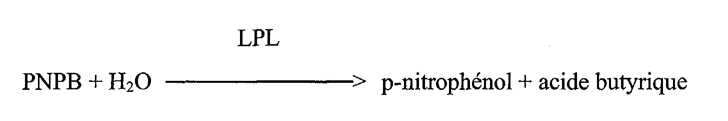

L'enzyme, à 20 U/ml, est incubée en présence du produit à l'essai et du PNPB, à 0,9 mM. La cinétique d'apparition du p-nitrophénol est réalisée à 37°C pendant 10 minutes. Elle est suivie par mesure de l'absorbance à 400 nm.

Les résultats sont consignés dans le tableau suivant, en pourcentage d'inhibition de l'activité de la lipoprotéine lipase par rapport au témoin.

| | Concentration d'incubation (en % ES) | Pourcentage d'inhibition de la LPL |
|---|---|---|
| Caféine | 0,01 | 0 % |
| Caféine | 0,1 | 0 % |
| Composition A | 0,01 | 5 % |
| Composition A | 0,1 | 40 % |

### F- Mesure de l'activité des métalloprotéinases MMP-2 et MMP-9

### (1) - Principe de la méthode

Les métalloprotéinases MMP-2 et MMP-9 sont rencontrées dans les adipocytes humains. A ce niveau, elles jouent un rôle dans la différenciation des pré-adipocytes en adipocytes et de ce fait dans les capacités de stockage du tissu adipeux.

L'objectif de ce test est d'évaluer les capacités d'inhibition d'un produit sur l'activité enzymatique des MMP-2 et MMP-9. Le test est fondé sur une réaction enzymatique entre la MMP-2 ou la MMP-9 purifiée et un substrat spécifique contenant un chromophore. La dégradation du substrat chromogènique par l'enzyme conduit à la production de fluorescence suivie par fluorimétrie.

### (2) -Protocole expérimental

**(i)** - Une MMP-9 recombinante humaine (CALBIOCHEM, PF024) est incubée, à 0,05 µg/ml, en présence du produit et du substrat chromogénique, la DQ-gélatine conjuguée à la fluorescéine (MOLECULAR PROBE, D12054) à 10 µg/ml. Le mélange réactionnel est incubé pendant une nuit à température ambiante, à l'abri de la lumière. La quantité de DQ-gélatine dégradée est mesurée par fluorescence (Ex : 355 nm ; Em : 460 nm).
**(ii) -** Une MMP-2 recombinante humaine (CALBIOCHEM, PF023) est incubée, à 0,1 µg/ml, en présence du produit et du substrat chromogénique, le DQ-gélatine conjuguée à la fluorescéine (MOLECULAR PROBE, D12054) à 10 µg/ml. Le mélange réactionnel est incubé pendant une nuit à température ambiante, à l'abri de la lumière. La quantité de DQ-gélatine dégradée est mesurée par fluorescence (Ex : 355 nm ; Em : 460 nm).

La réactivité du système enzymatique est systématiquement contrôlée pour la mesure de l'effet d'un inhibiteur de métalloprotéinase, la 1,10-phénanthroline. Les résultats sont consignés dans le tableau suivant, en pourcentage d'inhibition de l'activité enzymatique des MMP-2 et MMP-9 par rapport au témoin.

| | Concentration d'incubation (en % ES) | Effet inhibiteur de la MMP-2 | Effet inhibiteur de la MMP-9 |
|---|---|---|---|
| 1,10-phénanthroline | 0,0018 | 25 % | 75 % |
| Caféine | 0,1 | 0 % | 0 % |
| Composition A | 0,1 | 40 % | 35 % |

### G - Mise en jeu des récepteurs α₂ et β-adrénergiques (pour l'activité lipolytique)

### (1) - Principe de la méthode

Il s'agit de déterminer, dans un modèle "in vitro" d'adipocytes humains isolés, l'implication des récepteurs α₂ et/ou β-adrénergiques sur l'activité lipolytique du produit testé. La lipolyse est en effet sous le contrôle des récepteurs adrénergiques rencontrés à la surface des adipocytes. Deux types récepteurs co-existent :
- les récepteurs β-adrénergiques dont la stimulation induit la lipolyse
- les récepteurs α₂-adrénergiques dont la stimulation inhibe le lipolyse.

Un produit lipolytique peut mettre en jeu ces récepteurs soit par stimulation des récepteurs β-adrénergique (agoniste), soit par inhibition des récepteurs α₂-adrénergiques (antagoniste). La méthode décrite consiste en une incubation des produits en présence d'adipocytes humains en suspension, suivie d'une mesure de l'activité lipolytique par dosage des acides gras. L'incubation des adipocytes se fait en présence d'agents pharmacologiques, agonistes α₂-adrénergique ou antagonistes β-adrénergique, connus pour inhiber la lipolyse.

L'essai consiste à étudier l'effet du produit sur l'inhibition de la lipolyse induite par ces agents pharmacologiques.

### (2) - Protocole expérimental - résultats

### (i) - Modèle cellulaire :

Le test est réalisé à partir d'adipocytes humains isolés et préparés en suspension cellulaire. Les adipocytes sont isolés à partir du tissu adipeux abdominal sous-cutané récupéré lors d'opérations de chirurgies esthétiques (plasties abdominales) réalisées chez des femmes. Les cellules sont isolées à partir du tissu frais. Pour l'isolation des cellules, le tissu adipeux est isolé et dissocié par action d'une collagénase (SIGMA, 1 mg/ml, 30 minutes à 37°C, agitation douce). La collagènase digère le tissu conjonctif présent dans le tissu adipeux. Après digestion, les cellules sont filtrées et lavées dans un milieu de culture approprié contenant un milieu MEM sans rouge de phénol, sans glutamine (SIGMA) + 2,2 mg/ml de bicarbonate de sodium (GIBCO) + 50 UI de pénicilline (BIOWHITTAKER) + 50 µg/ml de streptomycine (BIOWHITTAKER) + 1% (v/v) de L-glutamine (BIOWHITTAKER) + 0,5% d'albumine sérique délipidée (SIGMA). La suspension d'adipocytes est utilisée immédiatement après sa préparation.

### (ii) - Incubation des produits avec les adipocytes :

Le produit est dilué dans le milieu de culture des adipocytes.

Le produit est incubé avec les cellules en suspension pendant deux heures à 37°C (250 µl de produit + 250 µl de suspension d'adipocytes).

Le produit est incubé sous trois conditions expérimentales :
- seul,
- en présence d'un agoniste α₂-adrénergique : la brimonidine à 10-9M (concentration préalablement déterminée pour inhiber d'environ 50% la lipolyse de base), et
- en présence d'un antagoniste β-adrénergique : le propranolol à 10⁻⁷ M (concentration préalablement déterminée pour inhiber d'environ 50% la lipolyse de base).

### (iii) - Paramètres d'évaluation :

A l'issue de l'incubation, la lyse cellulaire est contrôlée macroscopiquement par absence d'une couche lipidique à la surface des cellules.

Les milieux sous-nageants sont prélevés. Les acides gras non estérifiés sont dosés, par spectrophotométrie à l'aide d'un kit commercialisé, dans ces milieux sous-nageants (kit NEFA C, WAKO). Le dosage se fait contre une gamme étalon d'acides gras.

La réactivité des récepteurs α₂-adrénergiques et β-adrénergiques des adipocytes est systématiquement contrôlée pour la mesure de la levée de l'inhibition induite par les agents pharmacologiques, à savoir :
- pour l'inhibition induite par l'agoniste α₂-adrénergique, par l'utilisation de la phentolamine qui est un α₂-antagoniste
- pour l'inhibition induite par l'antagoniste β-adrénegique, par l'utilisation d'un agoniste β-adrénergique, l'isoprénaline.

Les résultats sont consignés dans les tableaux suivants en acides gras non estérifiés dans le sous-nageant (% par rapport au témoin)

| | Concentration d'incubation | dosage des acides gras non estérifiés dans le sous-nageant (% par rapport au témoin) |
|---|---|---|
| Témoin | - | 100 % |
| Brimonidine | 10⁻⁹ M | 0% |
| Composition A | 0,002% ES + Brimonidine 10⁻⁹ M | 100 % |
| Phentolamine | 10⁻⁷ M + Brimonidine 10⁻⁹ M | 79 % |

La composition A selon l'invention a donc un effet inhibiteur de l'effet anti-lipolytique d'un agoniste α₂-adrénergique.

| | Concentration d'incubation | dosage des acides gras non estérifiés dans le sous-nageant (% par rapport au témoin) |
|---|---|---|
| Témoin | - | 100 % |
| Propanolol | 10⁻⁷ M | 86 % |
| Composition A | 0,002% ES + Propanolol 10⁻⁷ M | 115 % |
| Isoprénaline | 10⁻⁵ M + Propanolol 10⁻⁷ M | 170 % |

La composition A selon l'invention a donc un effet inhibiteur de l'effet anti-lipolytique d'un antagoniste β-adrénergique.

### H - Mesure in vitro de la production d'acide hyaluronique

### (1) - Principe de la méthode

Les glycosaminoglycanes (GAGs) sont les protéoglycanes majoritaires du derme et jouent un rôle essentiel dans le maintien de l'intégrité de la peau. L'acide hyaluronique est un GAGs non sulfaté majoritaire qui joue un rôle essentiel dans l'hydratation de la peau par sa capacité à fixer jusqu'à 1000 fois son poids en eau. L'effet des produits sur le taux d'acide hyaluronique a été évalué par une technique in vitro.

### (2) - Protocole expérimental - Plan d'étude - résultats

Le taux d'acide hyaluronique est mesuré dans des cultures de fibroblastes dermiques humains normaux. Les cellules sont incubées, pendant 5 jours, en présence des produits solubilisés dans le milieu d'incubation. A l'issue de cette incubation, les milieux extracellulaires, dans lesquels l'acide hyaluronique est sécrété, sont prélevés. L'acide hyaluronique est coloré à l'aide d'un colorant spécifique, le STAINS ALL ((1-éthyl-2-[3-(1-éthylnaphtho-[1,2-d]thiazolin-2-ylidène)-2-méthylpropényl]naphtho-[1,2-d]thiazolium, bromide, SIGMA), qui interagit avec ce dernier pour produire un changement de spectre d'absorption entre 620 et 660 nm ; la mesure se fait par spectrophotométrie. Une gamme d'acide hyaluronique est réalisée en parallèle. 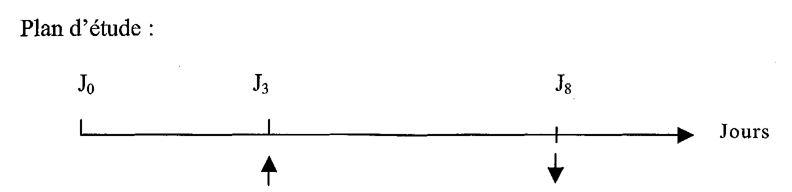
J0 : ensemencement des fibroblastes (plaques de culture de 24 puits, 15 300 cellules/puits)
J3 : incubation des produits testés, dilués dans le milieu d'incubation des fibroblastes
J8 : prélèvement des milieux d'incubation des fibroblastes, dosage de l'acide hyaluronique

Evaluation des effets : A l'issue des 5 jours d'incubation en présence des produits, les milieux d'incubation sont prélevés et incubés en présence du STAINS ALL. La réaction colorimétrique est révélée par addition d'eau. La quantification se fait par spectrophotométrie à 630 nm. Une gamme d'acide hyaluronique est réalisée en parallèle. Les résultats sont exprimés en acide hyaluronique extracellulaire par rapport au témoin.

| | Concentration d'incubation | Acide hyaluronique extracellulaire produit (% par rapport au témoin) |
|---|---|---|
| Témoin | - | 100 % |
| EGF | 10 ng/ml | 133 % |
| Composition A | 0,001 % ES | 130 % |
| Caféine | 0,001 % ES | 131 % |

### I - Mesure de l'activité de la hyaluronidase

### (1) - Principe de la méthode

L'objectif de ce test est de mesurer l'activité de la hyaluronidase. Cette enzyme, naturelle rencontrée dans la peau, dégrade l'acide hyaluronique. L'étude est réalisée in vitro sur enzyme purifiée.

### (2) - Protocole expérimental - résultats

La hyaluronidase (SIGMA), purifiée à partir de testicules bovins, est incubée à 0,01 U/ml en présence d'acide hyaluronique à 0,02 mg/ml, avec et sans produit à l'essai. L'incubation se fait pendant 60 minutes à 37°C.
Evaluation des effets : Après l'incubation, l'acide hyaluronique non dégradé par l'enzyme est précipité par un mélange acétate de sodium 24 mM/acide acétique 79mM (10 minutes, température ambiante). Le précipitât est mesuré au spectrophotomètre à 590 nm.
L'effet des produits à l'essai est comparé à celui observé en présence du glycyrrhizinate dipotassium, un inhibiteur de référence de la hyaluronidase.

| | Concentration d'incubation (en % ES) | Pourcentage d'inhibition de l'activité PDE 3 : |
|---|---|---|
| Glyccyrhizinate di K | 0,001 | 20 % |
| Glyccyrhizinate di K | 0,1 | 45 % |
| Caféine | 0,001 | 15 % |
| Caféine | 0,01 | 20 % |
| Composition A | 0,001 | 20 % |
| Composition A | 0,01 | 50 % |

### II - Exemples de formulation cosmétiques

Dans les exemples suivants les proportions sont exprimées en pourcentages pondéraux.

### Exemple 1 : Lait corporel amincissant

| | |
|---|---|
| MONTANOV^{TM} L | 3,00 % |
| Phytosqualane | 8,00 % |
| Huile d'amandes douces | 2,00 % |
| | |
| Eau | qsp 100 % |
| | |
| SEPIGEL^{TM} 501 | 1,50 % |
| | |
| Composition A' | 3,00 % |
| SEPICIDE^{TM} CI | 0,20 % |
| SEPICIDE^{TM} HB | 0,30 % |
| Parfum | 0,30 % |

### Exemple 2 : Crème anti-relâchement (cible ovale du visage)

| | |
|---|---|
| MONTANOV^{TM} 202 | 3,50 % |
| MONTANOV^{TM} 14 | 1,00 % |
| SEPILIFT^{TM} DPHP | 1,00 % |
| LANOL^{TM} 1688 | 15,00 % |
| Huile de germes de blé | 5, 00 % |
| | |
| Eau | qsp 100% |
| | |
| SIMULGEL^{TM} EG | 1,30 % |
| | |
| Composition A' | 2,00 % |
| SEPICIDE^{TM} CI | 0,20 % |
| SEPICIDE^{TM} HB | 0,30 % |
| Parfum | 0,10 % |

### Exemple 3 : Atomiseur anti-rondeurs

| | |
|---|---|
| MONTANE^{TM} 60 | 3,30 % |
| MONTANOX^{TM} 60 | 1,70 % |
| Caprilic / capric triglycérides | 6,00 % |
| Isohexadecane | 5,00 % |
| | |
| Magnésium Aluminium Silicate | 1,50 % |
| Eau | qsp 100 % |
| | |
| SIMULGEL^{TM} EG | 1,00 % |
| | |
| Composition A' | 2,00 % |
| Centella asiatica / extrait d'hydrocotyles | 1,00 % |
| SEPICIDE^{TM} CI | 0,20 % |
| SEPICIDE^{TM} HB | 0,30 % |
| Parfum | 0,40 % |
| Eau | qsp 100% |

### Exemple 4 : Gel amincissant fraîcheur

| | |
|---|---|
| SEPIGEL^{TM} 305 | 3,50 % |
| Hydroxyéthyl cellulose | 1,00 % |
| Caféine | 5,00 % |
| Menthol | 0,30 % |
| Ethanol | 50,00 % |
| Composition A' | 3,00 % |
| SEPICIDE^{TM} LD | 1,00 % |
| Parfum | 0,20 % |
| Eau | qsp 100 % |

### Exemple 5 : Fluide corporel minceur

| | |
|---|---|
| SIMULGEL^{TM} NS | 2,50 % |
| Gomme de xanthane | 0,20 % |
| LANOL^{TM} 99 | 5,00 % |
| Composition A' | 2,00 % |
| Extrait de Ginkgo Biloba | 2,00 % |
| Extrait de cola | 1,00 % |
| Extrait de Ginseng | 0,50 % |
| SEPICIDE^{TM} HB | 1,50 % |
| Parfum | 0,10 % |
| Eau | qsp 100 % |

### Exemple 6 : Lotion revitalisante fermeté destinée à être imprégnée sur des lingettes corporelles

| | |
|---|---|
| Composition A' | 1,50 % |
| Glycérine | 5,00 % |
| Ethanol | 5,00 % |
| Extrait de Ruscus | 3,00 % |
| SEPITONIC^{TM} M3 | 1,00 % |
| | |
| SEPICIDE^{TM} CI | 0,20 % |
| SEPICIDE^{TM} HB | 0,30 % |
| Eau | qsp 100 % |

### Exemple 7 : gel douche amincissant

| | |
|---|---|
| MONTALINE^{TM} C40 | 8,00 % |
| PROTEOL^{TM} OAT | 5,00 % |
| Sodium lauryl sulfate | 9,00 % |
| Composition A' | 3,00 % |
| Extrait de thé vert | 1,00 % |
| KATHON^{TM} CG | 0,80 % |
| | |
| | |
| Colorant vert | qs |
| Parfum de thé vert | 1,00 % |
| Acide lactique | qs PH=6,5 |
| Eau | qsp 100% |

### Exemple 8 : Massage désinfiltrant biphasique

| | |
|---|---|
| Huile de café arabica | 1,00 % |
| LANOL^{TM} 189 | 20,00 % |
| LANOL^{TM} 99 | 10,00 % |
| Huile de bourrache | 2,00 % |
| Parfum | 0,10 % |
| | |
| Composition A' | 3,00 % |
| Glycérine | 3,00 % |
| Ethanol | 10,00 % |
| Colorant bleu | qs |
| Eau | qsp 100 % |

Les définitions des produits commerciaux utilisés dans les exemples sont les suivantes :
SEPILIFT^{TM} DPHP : (Dénomination INCI : Dipalmitoyl hydroxyproline), commercialisé par la société SEPPIC ;
SEPICIDE^{TM} CI : Imidazoline urée, (agent conservateur) commercialisé par la société SEPPIC ;
SEPICIDE^{TM} HB : Mélange de phénoxyéthanol, de méthylparaben, d'éthylparaben, de propyl-paraben et de butylparaben, (agent conservateur) commercialisé par la société SEPPIC ;
SEPICIDE^{TM} LD : phénoxyéthanol commercialisé par la société SEPPIC ;
KATHON^{TM} CG : (Dénomination INCI : méthyl isothiazolinone / Méthyl chloroisothiazolinone) ;
MONTANE^{TM} 60 : Sorbitan stéarate ;
MONTANOX^{TM} 60 : Polysorbate 60 ;
SIMULGEL^{TM} EG : Latex inverse auto-inversible de copolymère tel que ceux décrits dans la publication internationale WO 99/36445 (dénomination INCI : Sodium acrylate/Sodium acryloyldimethyl taurate copolymer et Isohexadecane et Polysorbate 80 ) commercialisé par la société SEPPIC ;
SIMULGEL^{TM} NS : Latex inverse auto-inversible de copolymère tel que ceux décrits dans la publication internationale WO 99/36445 (dénomination INCI : hydroxyethylacrylate/Sodium acryloyldimethyl taurate copolymer et squalane et Polysorbate 60 ) commercialisé par la société SEPPIC ;
SEPIGEL^{TM} 305 : Latex inverse auto-inversible (dénomination INCI : Polacrylamide / C13-14 Isoparaffin / Laureth-7) ;
SEPIGEL^{TM} 501 : Latex inverse auto-inversible dénomination INCI : C13-14 Isoparaffin/Mineral Oil/Sodium polyacrylate/Polyacrylamide/Polysorbate 85) commercialisé par la société SEPPIC ;
LANOL^{TM} 99 : Isononanoate d'isononyle commercialisé par la société SEPPIC ;
LANOL^{TM} 189: Isostearyl isononanoate
LANOL^{TM} 1688 : Ethylhexanoate de cétéaryle commercialisé par la société SEPPIC ;
SEPITONIC^{TM} M3 : Mélange d'aspartate de magnésium, de gluconate de cuivre et de gluconate de zinc commercialisé par la société SEPPIC ;
MONTALINE^{TM} C40 : Cocamidopropyl betainamide MEA chloride
PROTEOL^{TM} OAT : Acides aminés d'avoine N'lauroylé oat amino acids ;
MONTANOV^{TM} 14 : Myristyl alcohol / Myristyl glucoside ;
MONTANOV^{TM} L : Agent émulsionnant à base d'alcool en C14-C22 et d'alkyl polyglucoside en C12-C20 tel que ceux décrits dans la demande de brevet européen EP 0 995 487 ;
MONTANOV^{TM} 202 est un agent émulsionnant à base d'alcool arachidylique d'alcool béhènylique et d'arachidyl polyglucoside.

## Revendications

1. Utilisation d'un composé de formule (1) : 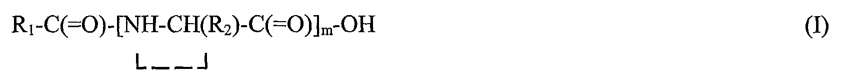 dans laquelle R₁ représente un radical hydrocarboné aliphatique, saturé ou insaturé, linéaire ou ramifié, comportant 11 atomes de carbone, R₂ représente la chaîne caractérisante d'un acide aminé et m est compris entre 1 et 50, ou d'un mélange desdits composés de formule (I), comme actif amincissant, dans une composition contenant un milieu cosmétiquement acceptable.

2. Utilisation telle que définie à la revendication 1, pour laquelle dans la formule (I), R₂ représente la chaîne caractérisante d'un acide aminé choisi parmi la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la glutamine, la cystéine, la cystine, la méthionine, l'hydroxy proline, l'hydroxy lysine, la sarcosine ou l'ornithine.

3. Utilisation telle que définie à la revendication 2, pour laquelle dans la formule (I), R₂ représente la chaîne caractérisante de la glycine, de l'alanine, de l'acide aspartique, de l'acide glutamique ou de la sarcosine.

4. Utilisation telle que définie à l'une des revendications 1 à 3, dans laquelle m est un nombre décimal compris entre 1 et 10 et est de préférence inférieur à 5.

5. Utilisation telle que définie à la revendication 4, dans laquelle m est un nombre décimal inférieur ou égal à 2 et est plus particulièrement inférieur ou égal à 1,4.

6. Utilisation telle que définie à l'une des revendications 1 à 5, dans laquelle m est égal à 1.

7. Utilisation telle que définie à l'une des revendications 1 à 6, de N-cocoyl aminoacides.

8. Procédé de traitement non thérapeutique du corps humain destiné à l'amincir, **caractérisé en ce que** l'on y applique une composition contenant un milieu cosmétiquement acceptable et une quantité efficace d'au moins un composé de formule (I) telle que définie à l'une des revendications 1 à 7.

9. Utilisation d'au moins un composé de formule (I), telle que définie à l'une des revendications 1 à 7, pour préparer un médicament à activité lipolytique, destiné à induire l'amincissement du corps humain.
